# EUROPEAN PATENT APPLICATION

(11) **EP 4 327 832 A1**
(43) Date of publication of application: **28.02.2024**
(21) Application number: 23193415.9
(22) Date of filing: 25.08.2023
(51) Int. Cl.: A61K 47/68, A61P 35/00, C07K 16/28

(54) **ANTI-SEZ6 ANTIBODY DRUG CONJUGATES**

(30) Priority: 26.08.2022 US 202263373668 P
(71) Applicant: AbbVie Inc., North Chicago, IL 60064 (US)
(72) Inventor: Faivre, Emily Jean, Lake Forest, IL, 60045 (US); Phillips, Andrew C., Libertyville, IL, 60048 (US); Reilly, Regina M., Libertyville, IL, 60048 (US); Ó Hainmhire, Eoghainín, Gurnee, IL, 60031 (US)
(74) Representative: Hoffmann Eitle

(57) **Abstract**

The present disclosure provides SEZ6 antibody drug conjugates (ADCs) comprising topoisomerase I inhibitors, including compositions and methods of using such ADCs.

## Description

### 1. CROSS REFERENCE TO RELATED APPLICATIONS

This application claims the benefit of U.S. provisional application ser. no. 63/373,668, filed August 26, 2022, which is hereby incorporated by reference in its entirety.

### 2. SEQUENCE LISTING

The instant application contains a Sequence Listing which has been submitted electronically and is hereby incorporated by reference in its entirety. Said Sequence Listing, created on August 4, 2023, is named SeqList_350794-46100 and is 14,965 bytes in size.

### 3. TECHNICAL FIELD

The present application pertains to anti-SEZ6 antibody drug conjugates (ADCs).

### 4. BACKGROUND

Seizure-related homolog 6 (SEZ6) is a transmembrane protein found on the cell surface of select neuronal lineage cells (Shimizu-Nishikawa K, et al. Cloning and characterization of seizure-related gene, SEZ6. Biochem Biophys Res Commun. 1995;216(1):382-9; Gunnersen JM, et al.). SEZ6 is selectively expressed on neuroendocrine tumors, including small cell lung cancer (SCLC); Kudoh S, et al. Significance of achaete-scute complex homologue 1 (ASCL1) in pulmonary neuroendocrine carcinomas; RNA sequence analyses using small cell lung cancer cells and ASCL1-induced pulmonary neuroendocrine carcinoma cells. Histochem Cell Biol. 2020;153(6):443-456). Small Cell Lung Cancer (SCLC) is an aggressive neuroendocrine carcinoma that represents 13-15% of all diagnosed lung cancers, with ~260,000 new cases diagnosed annually worldwide with ~33,000 diagnosed in the US leading to 25,000 deaths. The 5-year survival rate for extensive disease is 2% (-65% of patients), and the limited stage disease 5-year survival rate is 15%.

Conventional therapeutic treatments for cancer are often ineffective, thus there remains a need to develop more targeted and potent therapies for treating cancer. An antibody drug conjugate (ADC) comprising an antibody targeting SEZ6 conjugated to a cytotoxic drug via a chemical linker provides a targeted therapy for treating patients with tumors that express SEZ6.

### 5. SUMMARY

Provided here in is an anti-SEZ6 antibody drug conjugate of formula (IV): 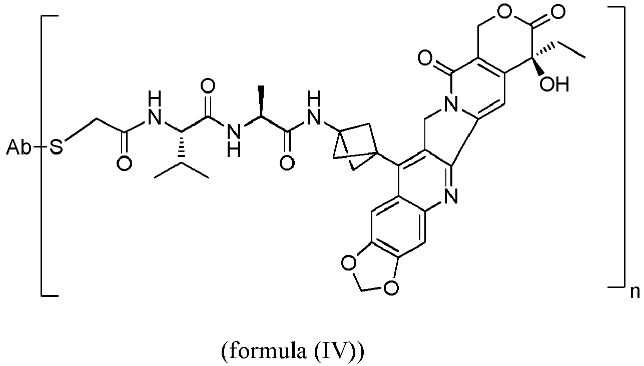 wherein n is an integer from 1 to 10, and wherein Ab is an anti-SEZ6 antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 3,
CDR-H2 has the amino acid sequence shown as SEQ ID NO: 4,
CDR-H3 has the amino acid sequence shown as SEQ ID NO: 5;
CDR-L1 has the amino acid sequence shown as SEQ ID NO: 6,
CDR-L2 has the amino acid sequence shown as SEQ ID NO: 7, and
CDR-L3 has the amino acid sequence shown as SEQ ID NO: 8.

In some embodiments, the anti-SEZ6 antibody of the antibody drug conjugate comprises (a) a heavy chain variable region having the amino acid sequence shown as SEQ ID NO: 1 and (b) a light chain variable region having the amino acid sequence shown as SEQ ID NO: 2.

In some embodiments, the anti-SEZ6 antibody of the antibody drug conjugate is an IgG1 antibody.

In some embodiments, the anti-SEZ6 antibody of the antibody drug conjugate comprises (a) a heavy chain having the amino acid shown as SEQ ID NO: 9 and (b) a light chain having the amino acid sequence shown as SEQ ID NO: 10.

In some embodiments, the anti-SEZ6 antibody drug conjugate has the structure of formula (IV): 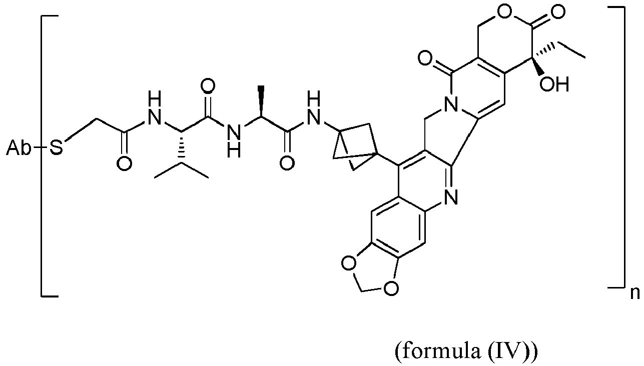 wherein n is an integer from 1 to 10, and wherein Ab is an anti-SEZ6 antibody comprising: two heavy chains each having an amino acid sequence shown as SEQ ID NO: 9 and two light chains each having an amino acid sequence shown as SEQ ID NO: 10.

In some embodiments, the anti-SEZ6 antibody drug conjugate has n of 6.

In some embodiments, the anti-SEZ6 antibody drug conjugate has n of 2.

In some embodiments, the anti-SEZ6 antibody drug conjugate has n of 4.

In some embodiments, the anti-SEZ6 antibody drug conjugate has n of 8.

In some embodiments, the anti-SEZ6 antibody drug conjugate has n of 10.

Also provided herein is a composition comprising a therapeutically effective amount of an anti-SEZ6 antibody drug conjugate disclosed herein, wherein the predominant species of the antibody drug conjugate in the composition has an n of 6.

Also provided herein is a pharmaceutical composition comprising a therapeutically effective amount of an anti-SEZ6 antibody drug conjugate disclosed herein and a pharmaceutically acceptable excipient, wherein the composition has a DAR of 6 or about 6

Also provided herein is a method of treating small cell lung cancer, wherein the method comprises administering a therapeutically effective amount of an anti-SEZ6 antibody drug conjugate disclosed herein to a patient in need thereof.

Also provided herein a method of treating small cell lung cancer, wherein the method of administering a therapeutically effective amount of a pharmaceutical composition disclosed herein comprising a therapeutically effective amount of an anti-SEZ6 antibody drug conjugate disclosed herein to a patient in need thereof.

### 6. BRIEF DESCRIPTION OF THE DRAWINGS

FIGS. 1A-1B show cell proliferation following administration of TOP1 inhibitor compounds for Calu-6 (lung adenocarcinoma; FIG. 1A) and A375 (malignant melanoma; FIG 1B) cell lines.
FIGS. 2A-2H show in vitro activity on SCLC cell lines of ADC-1, a negative control non-targeting ADC (ADC-2), or TOP1 inhibitor alone (Example 1G), on cell lines that were positive for SEZ6 mRNA: (FIG. 2A) H526, (FIG. 2B) H146, (FIG. 2C) H187, (FIG. 2D) H1963FP5, (FIG. 2E) CORL95, (FIG. 2H) H524; or on two cell lines that were negative for SEZ6 mRNA: (FIG. 2F) SBC5, and (FIG. 2G) SW1271.
FIGS. 3A-3D show dose response curves of ADC-1 treatment in H1963 (FIG. 3A) and H526 (FIG. 3B) cell-line derived xenograft (CDX) mouse models and LU95 (FIG. 3C) and LU149 (FIG. 3D) patient-derived xenograft (PDX) mouse models. fig

### 7. DETAILED DESCRIPTION

As used in this disclosure, the singular forms "a", "an" and "the" include plural referents unless the context clearly dictates otherwise. The terms "a" (or "an"), as well as the terms "one or more," and "at least one" can be used interchangeably herein unless the context clearly dictates otherwise.

As used in this disclosure and unless otherwise specified, the terms "about" and "approximately" generally refer to a range of numbers that one of skill in the art would consider equivalent to the recited value (i.e., having the same function or result). In many instances, the terms "about" and "approximately" may include numbers that are rounded to the nearest significant figure. In specific embodiments, the terms "about" and "approximately" shall be construed so as to allow normal variation as judged by a person of ordinary skill in the art, such as, for example, a variation within 20% or 10% or 5%. In specific embodiments, the terms "about" and "approximately" encompass the exact value recited. Unless the context clearly dictates otherwise, all numerical values provided herein are modified by the term about.

In certain embodiments, disclosed herein are anti-SEZ6 antibody-drug conjugates (ADCs), including anti-SEZ6 ADCs comprising TOP1 inhibitors, and methods of using the ADCs.

### 7.1. Topoisomerase 1 Inhibitors (TOP1i)

Topoisomerase 1 (TOP1) removes supercoils formed during DNA replication. TOP1 inhibitors (TOP1i) can bind and stabilize TOP1-DNA complexes, inducing DNA strand breakage and apoptosis.

In certain embodiments, provided herein is a topoisomerase I inhibitor drug ("TOP1i drug") according to structural formula (I), which may be purposed for targeted delivery to cells by conjugation to an anti-SEZ6 antibody.

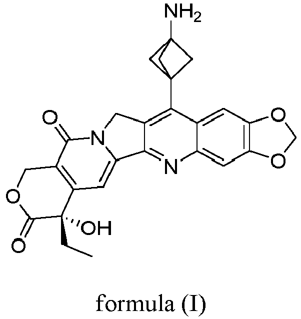

In certain embodiments, the TOP1i drug is (7*S*)-14-(3-aminobicyclo[1.1.1]pentan-1-yl)-7-ethyl-7-hydroxy-2*H*,10*H*-[1,3]dioxolo[4,5-*g*]pyrano[3',4':6,7]indolizino[1,2-*b*]quinoline-8,1(7*H*,13*H*)-dione.

In certain embodiments, the TOP1i drug as contemplated herein may be conjugated to an antibody via a linker as shown in structural formula (II): 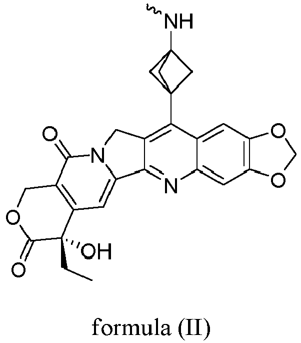 wherein 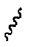 represents the point of attachment of a linker to the TOP1i drug.

### 7.1.1. Linker Drug LD1 (Structural Formula (III))

In certain embodiments, a TOP1i linker drug (LD1) is a compound according to formula (III). In certain embodiments, LD1 is (2*S*)-2-(2-bromoacetamido)-*N*-[(2*S*)-1-({3-[(7*S*)-7-ethyl-7-hydroxy-8,11-dioxo-7,8,11,13-tetrahydro-2*H*,10*H*-[1,3]dioxolo[4,5-g]pyrano[3',4' : 6,7]indolizino[1,2-*b*]quinolin-14-yl] bicyclo[1.1.1]pentan-1-yl}amino)-1-oxopropan-2-yl]-3-methylbutanamide.

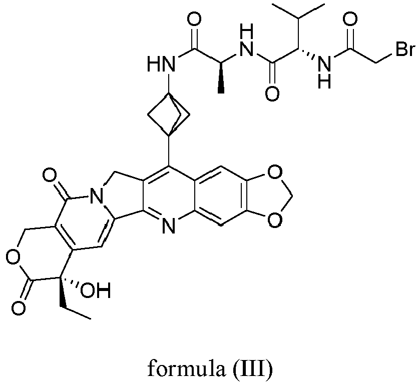

### 7.2. ANTI-SEZ6 ADCs

In certain embodiments, the TOP1i drug as described herein (i.e., formula (I)) may be conjugated to an anti-SEZ6 antibody to form an anti-SEZ6 TOP1i antibody drug conjugate. Antibody-drug conjugates selectively deliver one or more drug moiety(s) to target tissues, such as SEZ6 expressing tumors. Thus, in certain embodiments, the present disclosure provides anti-SEZ6 TOP1i ADC for therapeutic use in the treatment of small cell lung cancer.

In certain embodiments, TOP1i drugs are conjugated to the anti-SEZ6 antibody by way of a linker moiety. In certain embodiments, the linkers connect the TOP1i drug to the anti-SEZ6 antibody by forming a covalent linkage to the TOP1i drug at one location and a covalent linkage to the antibody at another. In certain embodiments, the covalent linkages are formed by reaction between functional groups on the linker and functional groups on the TOP1i drug and the anti-SEZ6 antibody. In certain embodiments, the anti-SEZ6 ADC of the present disclosure is comprised of an anti-SEZ6 antibody conjugated to the TOP1i linker drug of formula (III).

In certain embodiments, "Ab" refers to an anti-SEZ6 antibody comprising a heavy chain having an amino acid sequence shown as SEQ ID NO: 9 and a light chain having an amino acid sequence shown as SEQ ID NO: 10. In certain embodiments, Ab is conjugated to the TOP1i linker drug of formula (III).

### 7.2.1. Ab ANTI-SEZ6 Antibody

In certain embodiments, Ab is a humanized anti-SEZ6 IgG1 monoclonal antibody. In certain embodiments, Ab comprises variable regions and CDRs (complementary determining regions) identified according to rules developed in the art and/or by aligning sequences against a database of known variable regions.

Methods for identifying these regions are described in Kontermann and Dubel, eds., Antibody Engineering, Springer, New York, N.Y., 2001 and Dinarello et al., Current Protocols in Immunology, John Wiley and Sons Inc., Hoboken, N.J., 2000. For example, CDRs may be identified in accordance with one of the schemes provided by Kabat et al. (1991) Sequences of Proteins of Immunological Interest (5th Ed.), U.S. Dept. of Health and Human Services, PHS, NIH, NIH Publication No. 91-3242 (referred to herein as "Kabat"); or in accordance with AbM (Oxford Molecular/MSI Pharmacopia) (referred to herein as "AbM"). AbM can be obtained from the Abysis database at www.bioinf.org.uk/abs (maintained by A.C. Martin in the Department of Biochemistry & Molecular Biology University College London).

In certain embodiments, Ab comprises a heavy chain having an amino acid sequence shown as SEQ ID NO: 9 (variable region is **bold** and has an amino acid sequence shown as SEQ ID NO: 1; constant region is *italicized;* CDRs are underlined and have amino acid sequences shown as SEQ ID NOs: 3, 4 and 5 respectively, in order of appearance):

EVQLVQSGAEVKKPGESLKISCKGSGYSFTSSWINWVRQMPGKGLEWMGRIYP
GEGDTNYNGNFEGQVTISADKSISTAYLQWSSLKASDTAMYYCTRGLVMDYWG
QGTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSSDKTHTCPPCPA
PELLGGPSVFLFPPKPKDTLMISRTPEVTCVVVDVSHEDPEVKFNWYVDGVEVHNAKTKP
REEQYNSTYRVVSVLTVLHQDWLNGKEYKCKVSNKALPAPIEKTISKAKGQPREPQVYTLP

PSRDELTKNQVSLTCLVKGFYPSDIAVEWESNGQPENNYKTTPPVLDSDGSFFLYSKLTVD
KSRWQQGNVFSCSVMHEALHNHYTQKSLSLSPG (SEQ ID NO: 9).

In certain embodiments, Ab comprises a light chain having an amino acid sequence shown as SEQ ID NO: 10 (variable region is **bold** and has an amino acid sequence shown as SEQ ID NO: 2; constant region is *italicized;* CDRs are underlined and have amino acid sequences shown as SEQ ID NOs: 6, 7 and 8 respectively, in order of appearance):

EIVLTQSPATLSLSPGERATLSCRASQSVDYNGISYMHWYQQKPGQAPRLLIYA
ASNVQSGIPARFSGSGSGTDFTLTISSLEPEDFAVYYCQQSIEDPPTFGGGTKVEI
KRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC (SEQ ID NO: 10).

In certain embodiments, Ab comprises two heavy chains each having an amino acid sequence shown as SEQ ID NO: 9 and two light chains each having an amino acid sequence shown as SEQ ID NO: 10.

In certain embodiments, Ab comprises a CDR-H1 having an amino acid sequence shown as SEQ ID NO: 3; a CDR-H2 having an amino acid sequence shown as SEQ ID NO: 4; a CDR-H3 having an amino acid sequence shown as SEQ ID NO 5; a CDR-L1 having an amino acid sequence shown as SEQ ID NO: 6; a CDR-L2 having an amino acid sequence shown as SEQ ID NO 7; and a CDR-L3 having an amino acid sequence shown as SEQ ID NO 8. The amino acid sequence of CDR-H1 was identified using the AbM definition, and the remaining CDR amino acid sequences were identified using the Kabat definition.

In certain embodiments, Ab comprises a heavy chain variable domain having an amino acid sequence shown as SEQ ID NO: 1 and a light chain variable domain having an amino acid sequence shown as SEQ ID NO: 2.

### 7.2.2. Number of Linked Drugs

In certain embodiments, the ADCs disclosed herein comprise drug molecules linked to antibody moieties in various stoichiometric molar ratios depending on the configuration of the antibody and, at least in part, the method used to effect conjugation.

The terms "drug load" or "drug loading" refer to the number of drug molecules per antibody in an individual ADC molecule. The number of TOP1i drugs linked to an anti-SEZ6 ADC may vary and will be limited by the number of available attachments sites on the anti-SEZ6 antibody. As contemplated for the anti-SEZ6 ADCs of the invention, the linker will link a single TOP1i drug to the antibody in an anti-SEZ6 ADC. As long as the anti-SEZ6 ADC does not exhibit unacceptable levels of aggregation under the conditions of use and/or storage, anti-SEZ6 ADCs (i.e., formula (IV)) having an n of up to 10 are contemplated. In certain embodiments, the anti-SEZ6 ADCs have an n of in the range of from 1-10. In certain embodiments, the anti-SEZ6 ADCs have an n selected from 1, 2, 3, 4, 5, 6, 7, 8, 9, or 10. In certain embodiments, n is 2, 3, 4, 5 or 6. In certain embodiments, n is 2, 4, 6, 8, or 10. In certain embodiments, n is 2, 4, or 6. In certain embodiments, n is 6. In certain embodiments, the drug loading may comprise 1 drug molecule, 2 drug molecules, 3 drug molecules, 4 drug molecules, 5 drug molecules, 6 drug molecules, 7 drug molecules, 8 drug molecules, 9 drug molecules, or 10 drug molecules.

Provided herein is a conjugation method of producing an anti-SEZ6 ADC composition with the predominant species of ADC having an n of 6, and with the composition having a drug-antibody ratio (DAR) of 6 or about 6. The DAR is the average number of drugs linked to each antibody in the composition. Other methods of conjugation are known in the art and can be utilized to produce ADCs having different numbers of conjugated drugs (with an n of, e.g., 2, 3, 4, 5, 6 or 7) and compositions of different DARs (e.g., a DAR of 4, 5, 6 or about 4, about 5 or about 6).

### 7.2.3. Exemplary ADCs

In certain embodiments, an anti-SEZ-6 antibody drug conjugate of formula (IV) is provided: 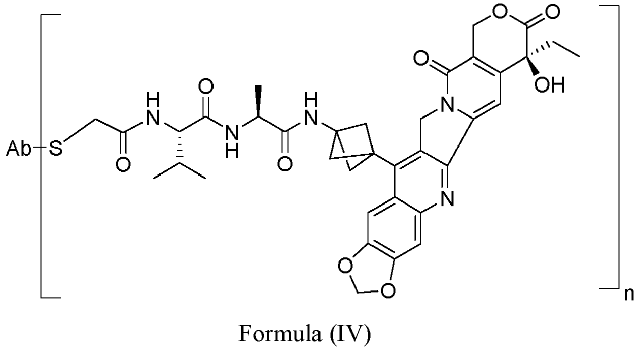 wherein antibody Ab comprises two heavy chains each having an amino acid sequence shown as SEQ ID NO:9 and two light chains each having an amino acid sequence shown as SEQ ID NO: 10. In certain embodiments, conjugation of the linker-drug to the antibody is via a linkage formed with a sulfhydryl group of a cysteine residue of the antibody. In certain embodiments, n has a value of 1, 2, 3, 4, 5, 6, 7, 8, 9, or 10.

In certain embodiments, n has a value of 2, 4, 6, 8, or 10. In certain embodiments, n is 6. In certain embodiments, the anti-SEZ6 ADC of structural formula (IV) comprises an Ab having a heavy chain having an amino acid sequence shown as SEQ ID NO: 9 and a light chain having an amino acid sequence shown as SEQ ID NO: 10 and n has a value of 2, 4, 6, 8 or 10. In certain embodiments, the anti-SEZ6 ADC of structural formula (IV) comprises an Ab having a heavy chain having an amino acid sequence shown as SEQ ID NO: 9 and a light chain having an amino acid sequence shown as SEQ ID NO: 10 and n has a value of 6.

As used herein, "ADC-1" is an ADC composition comprising ADCs according to structural formula (IV), wherein the Ab has two heavy chains each having an amino acid sequence shown as SEQ ID NO: 9 and two light chains each having an amino acid sequence shown as SEQ ID NO: 10 and a drug-antibody ratio (DAR) of about 6, with the predominant species having an n of 6. A procedure used to make ADC-1 is described in the Examples below. In an embodiment, ADC-1 is a composition comprising a plurality of ADC-1 species with two or more different values of n. In some embodiments, the composition of ADC-1 has an average drug-antibody ratio (DAR) of about 5.5, about 5.6, about 5.7, about 5.8, about 5.9, about 6.0, about 6.1, about 6.2, about 6.3, about 6.4, or about 6.5. In some embodiments, the composition of ADC-1 has an average of six molecules of the topoisomerase 1 inhibitor (Top1i) conjugated per anti-SEZ6 antibody. In some embodiments, the predominant species of ADC-1 in the composition has n = 6. In some embodiments, ADC-1 has an average DAR of about 6.

### 7.3. Methods of Use

In certain embodiments, methods for treating small cell lung cancer (SCLC) comprising administering to a subject in need thereof a therapeutically effective amount of an ADC of formula (IV) are provided.

The term "subject," as used herein, refers to a human. The terms "human," "patient," and "subject" are used interchangeably herein.

The terms "treat," "treating," and "treatment," as used herein, refer to a method of alleviating or abrogating a disease and/or its attendant symptoms.

The phrase "therapeutically effective amount" refers to an amount of an ADC sufficient to prevent the development of or to alleviate to some extent one or more of the symptoms of the condition or disorder being treated when administered for treatment in a particular subject or subject population.

### 8. EXAMPLES

The following Examples, which highlight certain features and properties of the exemplary embodiments of the ADCs, antibodies, and TOP1i as described herein, are provided for purposes of illustration.

### Example 1

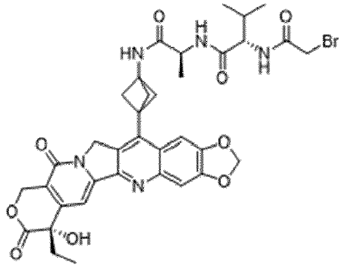

### Example 1

### (2S)-2-(2-bromoacetamido)-N-[(2S)-1-({3-[(7S)-7-ethyl-7-hydroxy-8,11-dioxo-7,8,11,13-tetrahydro-2H,10H-[1,3]dioxolo[4,5-g]pyrano[3',4':6,7]indolizino[1,2-b]quinolin-14-yl]bicyclo[1.1.1]pentan-1-yl}amino)-1-oxopropan-2-yl]-3-methylbutanamide

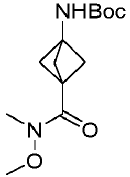

### Example 1A

### tert-butyl (3-(methoxy(methyl)carbamoyl)bicyclo[1.1.1]pentan-1-yl)carbamate

To a solution of 3-((*tert*-butoxycarbonyl)amino)blcyclo[1.1.1]pentane-1-carboxylic acid (4.9 g), *N*,*O*-dimethylhydroxylamine hydrochloride (2.2 g) and *N,N-*diisopropylethylamine (11.30 mL) in dichloromethane (10 mL) was added 1-[bis(dimethylamino)methylene]-1*H*-1,2,3-triazolo [4,5-*b*]pyridinium 3-oxide hexafluorophosphate (8.61 g) in portions at 10 °C. The reaction mixture was stirred at 20 °C for 12 hours. Two additional reactions were set up and allowed to stir at 20 °C for 12 hours as described. All three reactions were combined. The reaction was diluted with dichloromethane (200 mL) and added to 1 N aqueous HCl (50 mL). The precipitate formed was filtered, and the filtrate was allowed to separate. The organic layer was washed with saturated aqueous sodium bicarbonate solution (50 mL) and brine (50 mL), dried over anhydrous sodium sulfate, filtered, and the filtrate was concentrated under reduced pressure. The residue was purified by chromatography on silica gel, eluting with 1-50% ethyl acetate in petroleum ether to give the title compound. ¹H NMR (400 MHz, CDCl₃) *δ* ppm 4.97 (br s, 1H), 3.66 (s, 3H), 3.18 (s, 3H), 2.34 (s, 6H), 1.45 (s, 9H). MS (ESI+) *m*/*z* 271.2 (M+H)⁺.

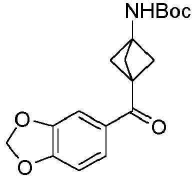

### Example 1B

### tert-butyl (3-(benzo[d][1,3]dioxole-5-carbonyl)bicyclo[1.1.1]pentan-1-yl)carbamate

To a solution of 5-bromobenzo[*d*][1,3]dioxole (8.83 g) in tetrahydrofuran (100 mL) was added n-butyllithium (17.57 mL, 2.5 M in hexane) slowly at -65 °C under nitrogen gas. The mixture was stirred at -65 °C for 30 minutes. A solution of Example 1A (4.75 g) in tetrahydrofuran (40 mL) was added slowly. The mixture was stirred at -65 °C for 3 hours. Three additional reactions were set up and allowed to stir at -65 °C for 3 hours. All four reactions were combined. The mixture was quenched with saturated aqueous ammonium chloride solution (500 mL) and extracted with ethyl acetate (3 × 500 mL). The combined organic layers were washed with brine (500 mL), dried over anhydrous sodium sulfate, filtered, and the filtrate was concentrated under reduced pressure. The residue was purified by chromatography on silica gel, eluting with 1-50% ethyl acetate in petroleum ether to give the title compound. ¹H NMR (400 MHz, CDCl₃) *δ* ppm 7.63 (dd, 1H), 7.45 (d, 1H), 6.82 (d, 1H), 6.03 (s, 2H), 5.04 (br s, 1H), 2.50 (s, 6H), 1.46 (s, 9H). MS (ESI+) *m*/*z* 354.2 (M+Na)⁺.

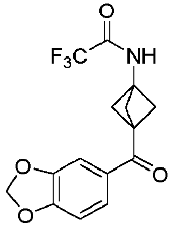

### Example 1C

### N-(3-(benzo[d][1,3]dioxole-5-carbonyl)bicyclo[1.1.1]pentan-1-yl)-2,2,2-trifluoroacetamide

Step 1: To a solution of Example 1B (6.2 g) in dichloromethane (62 mL) was added trifluoroacetic acid (62 mL) slowly at 0 °C. The reaction was stirred at 25 °C for 4 hours. Two additional reactions were set up and stirred at 25 °C for 4 hours. Each mixture was concentrated under reduced pressure. Each residue was used in the next step without further purification.

Step 2: To a solution of crude product above in dichloromethane (62 mL) was added A, A-di isopropylethylamine (16.34 mL) and trifluoroacetic anhydride (3.96 mL) dropwise at 0 °C. The mixture was stirred at 25 °C for 2 hours. Two additional reactions were set up as described and stirred at 25 °C for 2 hours. All three reactions were combined and poured into water (200 mL), extracted with dichloromethane (2 × 200 mL). The organic layer was washed with brine (200 mL), dried over anhydrous sodium sulfate, filtered, and the filtrate was concentrated under reduced pressure. The residue was purified by column chromatography on silica gel, eluting with 25% ethyl acetate in petroleum ether to give the title compound. ¹H NMR (501 MHz, CDCl₃) *δ* ppm 7.61 (dd, 1H), 7.43 (d, 1H), 7.00 (s, 1H), 6.85 (d, 1H), 6.05 (s, 2H), 2.62 (s, 6H). MS (ESI+) *m*/*z* 328.2 (M+H)⁺.

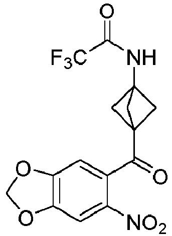

### Example 1D

### 2,2,2-trifluoro-N-(3-(6-nitrobenzo[d][1,3]dioxole-5-carbonyl)bicyclo[1.1.1]pentan-1-yl)acetamide

To a solution of Example 1C (4.3 g) in acetic anhydride (25 mL) was added copper(II) nitrate trihydrate (4.76 g) in portions at 0 °C. The mixture was stirred at 0 °C for 3 hours. Three additional reactions were set up and stirred at 0 °C for 3 hours as described. All four reactions were combined. The mixture was poured into water (50 mL) and extracted with ethyl acetate (5 × 100 mL). The organic layer was dried over anhydrous sodium sulfate, filtered, and the filtrate was concentrated under reduced pressure. The residue was purified by column chromatography on silica gel, eluting with 75% ethyl acetate in petroleum ether to give the title compound. ¹H NMR (400 MHz, CDCl₃) *δ* ppm 7.61 (s, 1H), 6.77 (br s, 1H), 6.64 (s, 1H), 6.21 (s, 2 H), 2.43 (s, 6 H). MS (APCI+) *m*/*z* 373.1 (M+H)⁺.

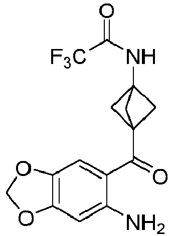

### Example 1E

### N-(3-(6-aminobenzo[d][1,3]dioxole-5-carbonyl)bicyclo[1.1.1]pentan-1-yl)-2,2,2-trifluoroacetamide

To a solution of Example 1D (4 g) in ethanol (40 mL) and water (8 mL), was added iron (5.4 g) and ammonium chloride (5.17 g) under nitrogen. The mixture was stirred at 100 °C for 3 hours. Three additional reactions were set up and stirred at 100 °C for 3 hours as described. After cooling to ambient temperature, all four reactions were combined. The mixture was poured into water (1 L) and extracted with ethyl acetate (5 × 500 mL). The combined organic phases were dried over anhydrous sodium sulfate, filtered, and the filtrate was concentrated under reduced pressure. The residue was purified by column chromatography on silica gel, eluting with 10-75% ethyl acetate in petroleum ether to give the title compound. ¹H NMR (400 MHz, CDCl₃) *δ* ppm 7.22 (s, 1H), 6.70 (s, 1H), 6.50 (s, 2H), 6.14 (s, 1H), 5.92 (s, 2H), 2.63 (s, 6H). MS (ESI+) *m*/*z* 343.2 (M+H)⁺.

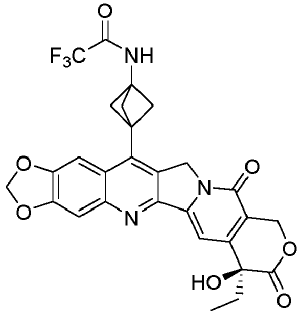

### Example 1F

### (S)-N-(3-(7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)bicyclo[1.1.1]pentan-1-yl)-2,2,2-trifluoroacetamide

To a suspension of Example 1E (3.5 g) and (*S*)-4-ethyl-4-hydroxy-7,8-dihydro-1*H-*pyrano[3,4-*ƒ*]indolizine-3,6,10(4*H*-trione (2.69 g) in toluene (140 mL) was added *para*toluenesulfonic acid monohydrate (1.945 g). The mixture was stirred at 115 °C for 12 hours. Three additional reactions were set up and stirred at 115 °C for 12 hours as described. After cooling to ambient temperature, all four reactions were combined. The mixture was filtered and the solid collected was triturated with acetonitrile (200 mL) to give the title compound. ¹H NMR (400 MHz, dimethyl sulfoxide *-d₆*) *δ* ppm 10.28 (s, 1H), 7.64 (s, 1H), 7.52 (s, 1H), 7.24 (s, 1H), 6.47 (s, 1H), 6.30 (dd, 2H), 5.42 (s, 2H), 5.36 (s, 2H), 2.87 (s, 6H), 1.94 - 1.77 (m, 2H), 0.88 (t, 3H). MS (ESI+) *m*/*z* 570.3 (M+H)⁺.

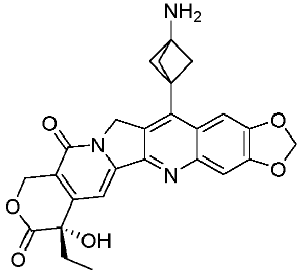

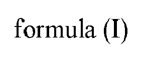

### Example 1G

### (7S)-14-(3-aminobicyclo[1.1.1]pentan-1-yl)-7-ethyl-7-hydroxy-2H,10H-[1,3]dioxolo[4,5-g]pyrano[3',4':6,7]indolizino[1,2-b]quinoline-8,11(7H,13H)-dione

To a solution of Example 1F (3 g) in methanol (30 mL) was added HCl (60 mL, 4 M in methanol). The mixture was stirred at 65 °C for 4 hours. Four additional reactions were set up and stirred at 65 °C for 4 hours as described above. After cooling to ambient temperature, all five reactions were combined. The mixture was concentrated under reduced pressure, and the residue was triturated with methanol (200 mL) to give the title compound. ¹H NMR (400 MHz, dimethyl sulfoxide *-d₆*) *δ* ppm 9.23 (s, 3H), 7.54 (s, 1H), 7.51 (s, 1H), 7.24 (s, 1H), 6.30 (d, 2H), 5.41 (s, 2H), 5.39 - 5.26 (m, 2H), 2.79 (s, 6H), 1.87 (hept, 2H), 0.88 (t, 3H). MS (ESI+) *m*/*z* 474.3 (M+H)⁺.

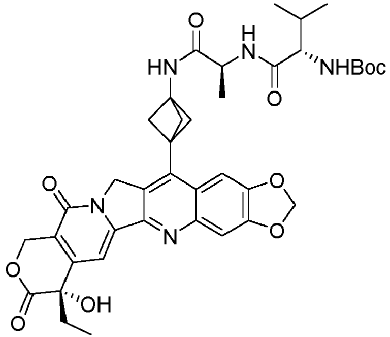

### Example 1H

### tert-butyl ((S)-1-(((S)-1-((3-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)bicyclo[1.1.1]pentan-1-yl)amino)-1-oxopropan-2-yl)amino)-3-methyl-1-oxobutan-2-yl)carbamate

To a suspension of (*S*)-2-((*S*)-2-((*tert*-butoxycarbonyl)amino)-3-methylbutanamido)propanoic acid (2.49 g), 2-hydroxypyridine 1-oxide (1.31 g), and *N*¹-((ethylimino)methylene)-*N*³,*N*³-dimethylpropane-1,3-diamine hydrochloride (2.26 g) in acetonitrile (40 mL), was added 2,6-lutidine (2.74 mL). The mixture was stirred at ambient temperature for 30 minutes. In a separate flask, Example 1G (4 g) and 2,6-lutidine (2.74 mL) were combined in *N*,*N*-dimethylformamide (40 mL) and the above solution was added. The mixture was stirred at ambient temperature overnight. The mixture was concentrated under reduced pressure and the residue was dissolved in dichloromethane (300 mL), washed with saturated aqueous ammonium chloride solution (100 mL), brine (100 mL), dried over anhydrous sodium sulfate, filtered, and the filtrate was concentrated under reduced pressure. The residue was purified by column chromatography on silica gel, eluting with 0-10% methanol in dichloromethane to give the title compound. ¹H NMR (400 MHz, dimethyl sulfoxide *-d₆*) *δ* ppm 8.65 (s, 1H), 7.89 (d, 1H), 7.61 (s, 1H), 7.49 (s, 1H), 7.22 (s, 1H), 6.77 (d, 1H), 6.46 (s, 1H), 6.29 (d, 2H), 5.41 (s, 2H), 5.32 (s, 2H), 4.29 (q, 1H), 3.87 - 3.77 (m, 1H), 2.76 (s, 6H), 1.99 (q, 1H), 1.92 - 1.81 (m, 2H), 1.41 (s, 9H), 1.25 (d, 3H), 0.92 - 0.80 (m, 9H). MS (ESI+) *m*/*z* 744.4 (M+H)⁺.

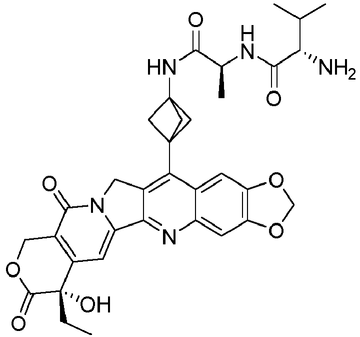

### Example 1I

### (5)-2-amino-N-((S)-1((3-((S)-7-ethyl-7-hydroxy-8,11-dioxo-8,10,11,13-tetrahydro-7H-[1,3]dioxolo[4,5-g]pyrano[3',4':6,7]indolizino[1,2-b]quinolin-14-yl)blcyclo[1.1.1]pentan-1-yl)amino)-1-oxopropan-2-yl)-3-methylbutanamide

Example 1H (5.5 g) was treated with trifluoracetic acid (30 mL) at ambient temperature for 30 minutes. The mixture was concentrated under reduced pressure and the residue was dissolved in 50% acetonitrile in water (200 mL). The solution was lyophilized to give the title compound. ¹H NMR (600 MHz, dimethyl sulfoxide *-d₆*) *δ* ppm 8.80 (s, 1H), 8.59 (d, 1H), 8.10 (d, 3H), 7.60 (s, 1H), 7.48 (s, 1H), 7.23 (s, 1H), 6.33 - 6.26 (m, 2H), 5.41 (d, 2H), 5.35 - 5.23 (m, 2H), 4.35 (p, 1H), 3.66 - 3.63 (m, 1H), 2.77 (s, 6H), 2.17 - 2.06 (m, 1H), 1.91 - 1.83 (m, 2H), 1.31 (d, 3H), 1.01 - 0.96 (dd, 6H), 0.89 (t, 3H). MS (ESI+) *m*/*z* 644.4 (M+H)⁺.

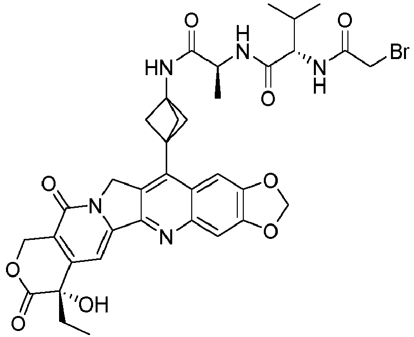

### Example 1J

### (2S)-2-(2-bromoacetamido)-N-[(2S)-1-({3-[(7S)-7-ethyl-7-hydroxy-8,11-dioxo-7,8,11,13-tetrahydro-2H,10H-[1,3]dioxolo[4,5-g]pyrano[3',4':6,7]indolizino[1,2-b]quinolin-14-yl]bicyclo[1.1.1]pentan-1-yl}amino)-1-oxopropan-2-yl]-3-methylbutanamide

To a solution of 2-bromoacetic acid (1.435 g) in *N*,*N*-dimethylformamide (26 mL) was added ethyl 2-ethoxyquinoline-1(2*H*)-carboxylate (2.55 g). The mixture was stirred at ambient temperature for 10 minutes. In a separate flask, Example 1I (4.5 g) and 2,6-lutidine (3.61 mL) were combined in *N*,*N*-dimethylformamide (26 mL), and the above solution was added. The mixture was stirred at ambient temperature for 30 minutes. The mixture was acidified with trifluoroacetic acid (4 mL) and purified by reverse-phase HPLC on a CombiFlash^{®} Teledyne Isco system using a Luna column (250 × 50 mm, 10 mm), eluting with 5-75% acetonitrile in water containing 0.1% trifluoroacetic acid over 30 minutes to give the title compound after lyophilization. ¹H NMR (600 MHz, dimethyl sulfoxide *-d₆*) *δ* ppm 8.57 (s, 1H), 8.32 (d, 1H), 8.16 (d, 1H), 7.67 (s, 1H), 7.52 (s, 1H), 7.24 (s, 1H), 6.30 (dd, 2H), 5.42 (s, 2H), 5.38 (d, 2H), 4.28 - 4.19 (m, 2H), 4.03 - 3.91 (m, 2H), 2.76 (s, 6H), 2.02 (h, 1H), 1.86 (ddp, 2H), 1.25 (d, 3H), 0.93 - 0.84 (m, 9H). MS (ESI+) *m*/*z* 764.46 (M+H)⁺.

### Example 2: Cell Proliferation

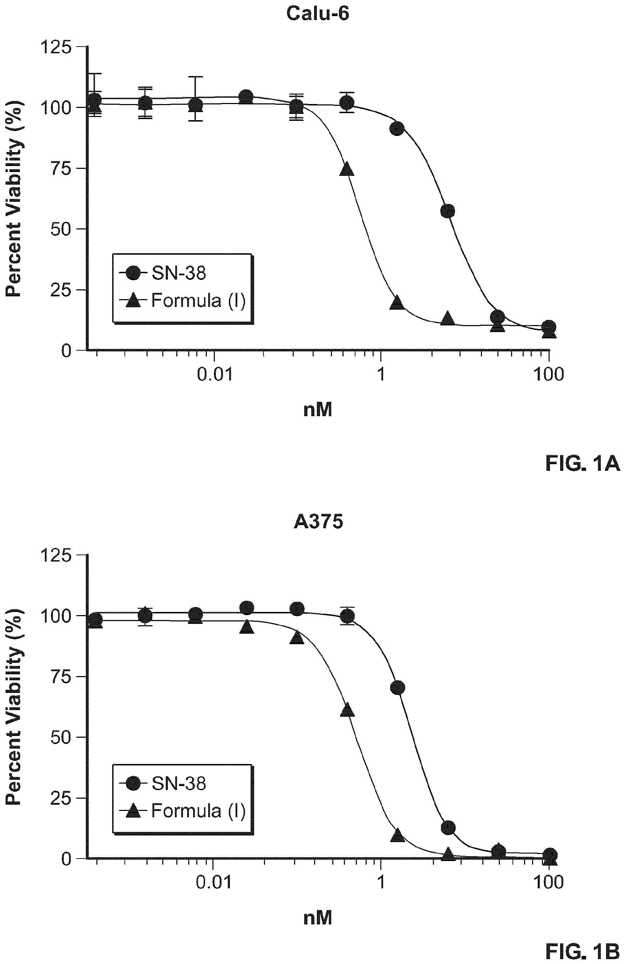

The effect of TOP1i drugs on cell viability was assessed by monitoring ATP presence for metabolically active cells. Results are shown in FIGS. 1A, 1B, and Table 1. Proliferation of Calu-6 cells (lung adenocarcinoma) and A375 cells (malignant melanoma) were quantified using CellTiter-Glo^{®} luminescence. As shown in FIG. 1A, 1B and in Table 1, the compound of formula (I) (Example 1G) exhibited sub-nanomolar potency for reducing viability of proliferating Calu-6 cells (0.57 nM, FIG. 1A) and A375 (0.50 nM, FIG. 1B). This sub-nanomolar potency was improved over that of the TOP1i drug SN-38 in the same assay, which demonstrated an EC₅₀ of 6.58 nM (Calu-6) and 2.30 nM (A375).

| **Table 1: TOP1i Compound Potency** | | |
|---|---|---|
| Compound | EC₅₀ (nM) | |
| | Calu-6 | A375 |
| SN-38 | 6.58 | 2.30 |
| Example 1G (formula 1) | 0.57 | 0.50 |

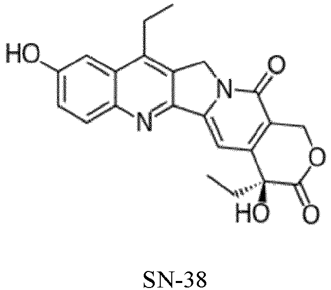

### Example 3: Preparation and Purification of Antibody

Antibody Ab was expressed in Chinese hamster ovary (CHO) cells as an IgG1 antibody with the heavy and light chain sequences of SEQ ID NOs: 9 and 10, respectively. Ab was isolated and purified prior to conjugation.

### Example 4: Procedure for Conjugation (ADC-1)

Ab was conjugated to a linker drug (Example 1J) as described below to form antibody drug conjugate ADC-1.

The Ab antibody from Example 3 was globally reduced with an excess of reducing agent and then conjugated with an excess of drug-linker. This results in the final drug product comprising an ADC primarily containing six drug molecules, i.e., an ADC of formula (IV) with an n of 6 and a DAR of 6 or about 6. The DAR of ADC-1 was determined by LC-MS. LC-MS analysis was performed using an Agilent 1100 HPLC system interfaced to an Agilent LC/MSD TOF 6220 ESI mass spectrometer. ADC-1 was reduced with 5 mM (final concentration) Bond-Breaker^{®} TCEP solution (Thermo Scientific, Rockford, IL), loaded onto a Protein Microtrap (Michrom Bioresorces, Auburn, CA) desalting cartridge, and eluted with a gradient of 10% B to 75% B in 0.2 minutes at ambient temperature. Mobile phase A was H₂O with 0.1% formic acid (FA), mobile phase B acetonitrile with 0.1% FA, and the flow rate was 0.2 mL/minute. Electrospray-ionization time-of-flight mass spectra of the co-eluting light and heavy chains were acquired using Agilent MassHunterTM acquisition software. The extracted intensity vs. m/z spectrum was deconvoluted using the Maximum Entropy feature of MassHunter software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC-1.

A solution of Ab antibody at 4 °C was treated with PBSE (potassium phosphate dibasic/sodium chloride/ethylenediaminetetraacetic acid buffer) (125 mM potassium phosphate, 150 mM NaCL, 6.3 mM EDTA, pH 7.2) to achieve a final concentration of 0.1 mM. Tris (2-carboxyethyl) phosphine (TCEP, 10 mM, 10 molar equivalents, Bond Breaker^{™}, Thermo Scientific^{™}) was added to the Ab antibody solution (approximately 10~20 mg/mL in 0.5X PBSE with gentle stir plate mixing (225 rpm) for 5 minutes. Following incubation at 4°C for 20 hours, the solution was brought to 22°C.

UFDF (ultrafiltration/difiltration) was applied to buffer exchange the reduced Ab antibody into PBSE buffer using a Pellicon^{®} 3 0.57 m² Cassette (Millipore). Ultrafiltration (UF) was performed until the Ab antibody reached a concentration of 40 mg/mL. Diafiltration (DF) was performed with 10 diavolumes (DV) of PBSE.

A 6.6 molar equivalents of 10 mM solution of linker drug (Example 1J) in *N,N-*dimethylacetamide with gentle stir plate mixing was incubated at 23 °C for 16 hours. Following incubation, 8.0 equivalents of 100 mM *N*-Acetyl-L-cysteine (NAC, Sigma Aldrich) prepared in sterile purified water, were added to the solution, gently mixed, followed by 2-hour incubation at 23 °C.

UFDF was applied to buffer exchange the resulting antibody drug conjugate (ADC-1) into a mixture of DMSO (dimethyl sulfoxide) and 15 mM histidine buffer, pH 6, and thereafter into a 15 mM histidine buffer using a Pellicon^{®} 3 0.57 m² Cassette (Millipore). Ultrafiltration and diafiltration were performed. The ADC-1 solution was collected then further diluted with buffer. The final ADC-1 solution was sterile filtered with a 0.22 µm filter and stored at -80 °C.

### Example 5A: Control ADC (ADC-2)

As used herein, ADC-2 is an ADC having an antibody, Ab2, conjugated to LD1 (formula (III), Example 1J). Ab2 is MSL109, which is a monoclonal antibody that binds to CMV glycoprotein H. MSL109 is used as a non-targeting control antibody/negative control antibody.

### Example 5B: Procedure for Conjugation (ADC-2)

Aqueous ethylenediaminetetraacetic acid disodium salt (EDTA, 0.5 M, 974 µL, Sigma Aldrich) was added to 243.3 mL of a purified solution of Ab2 (12.3 mg/mL in 20 mM Tris buffer, pH 7.4). Tris(2-carboxyethyl) phosphine (TCEP, 500 mM, 6.0 molar eq, 242.4 µL, Bond Breaker^{™}, Thermo Scientific^{™}) was added to the solution of Ab2 with 2 mM EDTA, and gently stirred and kept at 4 °C for 20 hours. Tris buffer at 4 °C (1.0 M, pH 8.0) was added to the solution (24.5 mL, 10% v/v). The linker-drug LD1 (Example 1J, (2*S*)-2-(2-bromoacetamido)-*N*-[(2*S*)-1-({3-[(7*S*)-7-ethyl-7-hydroxy-8,11-dioxo-7,8,11,13-tetrahydro-2*H*,10*H*-[1,3]dioxolo[4,5-*g*]pyrano[3',4':6,7]indolizino[1,2-*b*]quinolin-14-yl]bicyclo[1.1.1]pentan-1-yl}amino)-1-oxopropan-2-yl]-3-methylbutanamide) was added to the reduced antibody at pH 8.0 (10 equivalents, 22.5 mL of 10 mM solution of LD1, Example 1J, in *N*,*N*-dimethylacetamide, 90% purity). The conjugation was gently stirred and allowed to stand for 1.5 hours in an ambient temperature water bath. The conjugation was quenched with addition of 4 equivalents of an aqueous solution of *N*-acetyl-L-cysteine (NAC, Sigma Aldrich, 100 mM solution in 1X DPBS, 0.8 mL). The mixture was gently stirred and allowed to stand for 1 hour at ambient temperature. Tris buffer (15%, Sigma, 1.0 M, pH 7.4, 44 mL) was added to adjust the resulting ADC-2 solution to pH 7.5. The ADC-2 solution was stored at 4 °C overnight. The ADC-2 solution was concentrated, and buffer exchanged via ultrafiltration/diafiltration (Pellicon^{®} 3 0.22 m² Cassette (Millipore)). The final ADC-2 solution was filtered through a 0.22 µm filter and the resulting ADC-2 sample stored at 4 °C. Final volume of ADC-2 was 138 mL.

### Example 6: SEZ6 Expression and Cell Viability Assay In Vitro

SCLC cell lines NCI-H526 ("H526"), NCI-H524 ("H524"), NCI-H146 ("H146"), NCI-H1963 ("H1963") and SW-1271 were obtained from the American Type Culture Collection (ATCC). COR-L95 cells were obtained from Millipore Sigma (#96020733). SBC-5 cells were obtained from the Japanese Collection of Research Bioresources Cell Bank (JCRB).

SCLC cell lines (shown in Table 2, column 1) were plated in white 384-well plates (Corning^{®} 3765) at 1,000-2,000 cells/30 µl RPMI-1640 base media containing 10% heat-inactivated FBS (fetal bovine serum). Cell plates were incubated overnight at 37 °C in 5% CO₂. Using a Tecan D300e Digital Dispenser, a dose response using a stock concentration of ADC or solution containing Example 1G and DMSO (dimethyl sulfoxide) was applied to the cells on day 0 and incubated for an additional 10 days (ADCs) or 3 days (Example 1G) at 37 °C in 5% CO₂. Samples were run in duplicate. Cell viability was assessed with the CellTiter-Glo^{™} Luminescent Cell Viability Assay (Promega, #G7570) according to the manufacturer's instructions, with the exception that the plates were shaken for 30 minutes prior to reading. Luminescence was read on the BioTek Synergy Neo2, and data was analyzed using GraphPad Prism software.

### Results:

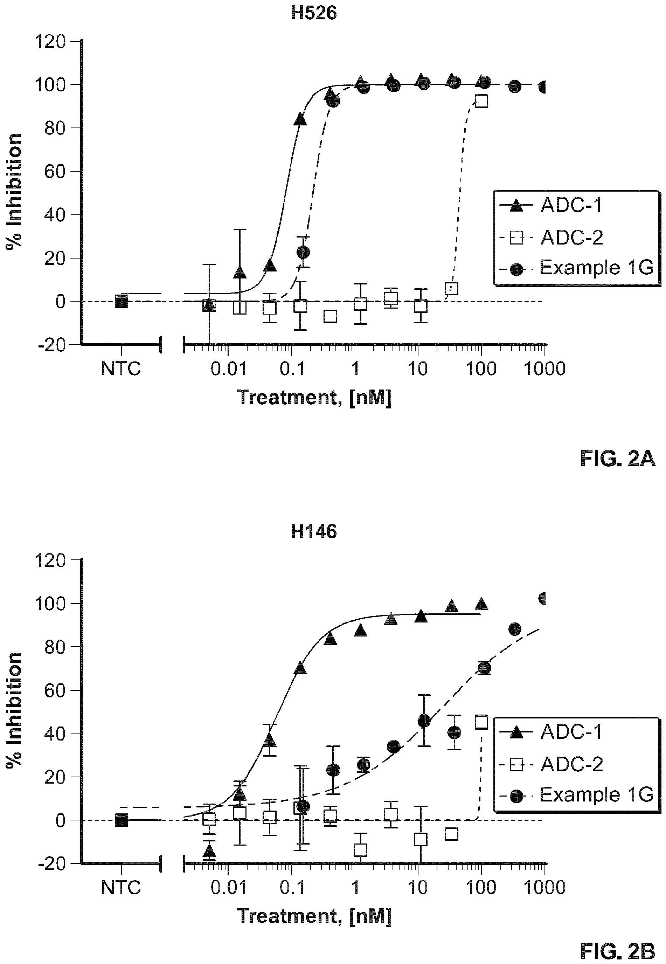

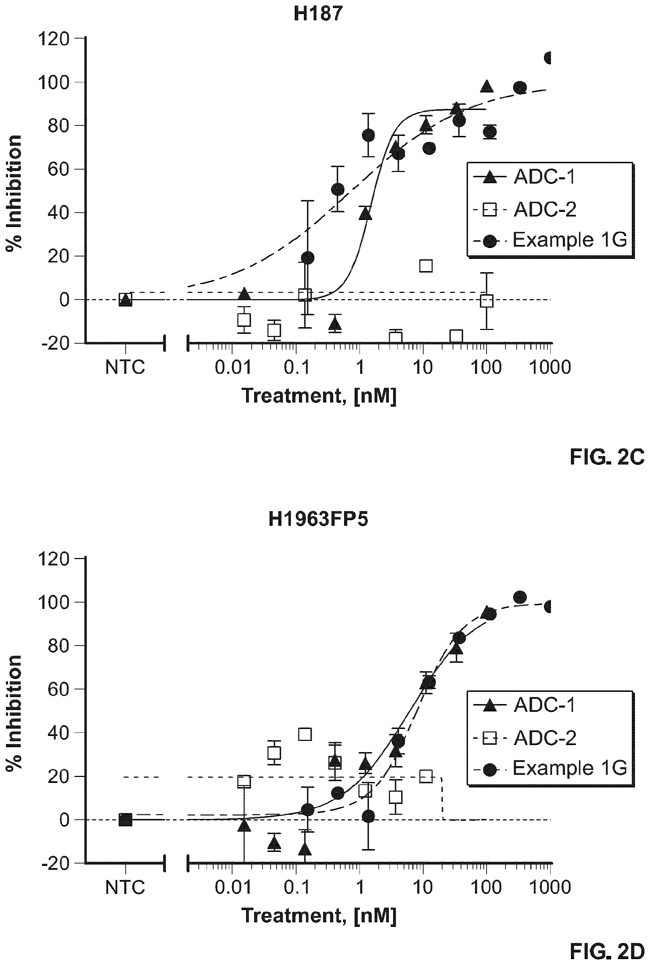

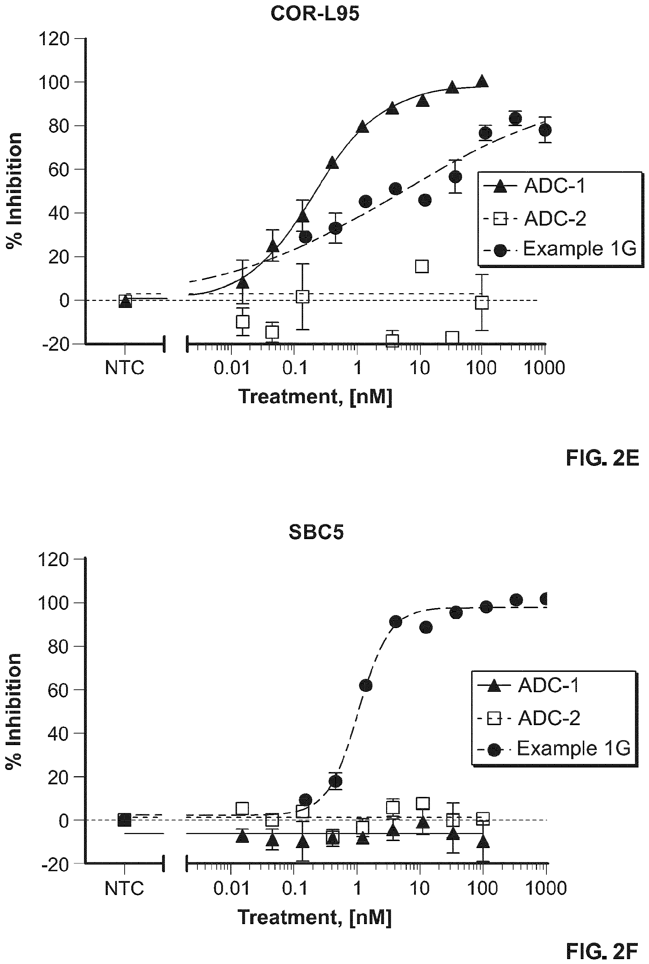

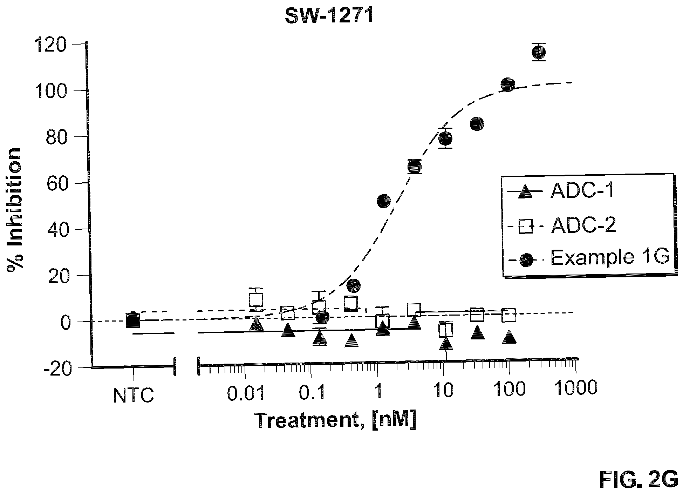

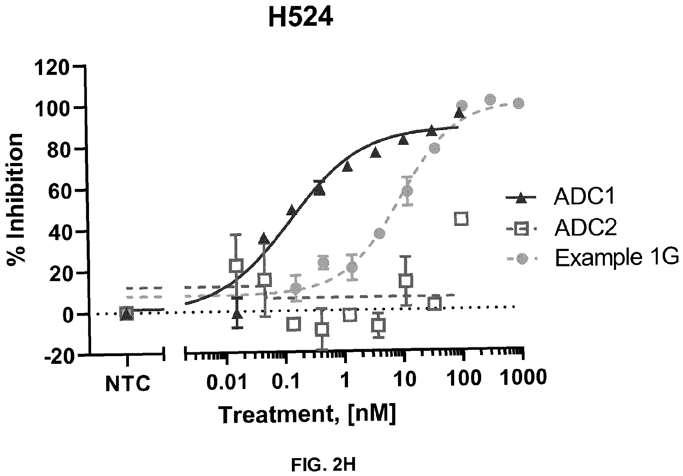

As shown in FIGS. 2A-2G and Table 2, ADC-1 targeting SEZ6 had potent activity, displaying sub- to low-nM growth inhibitory IC₅₀ against five SCLC cell lines that were positive for SEZ6 mRNA (NCI-H524, NCI-H526, COR-L96, NCI-H146, NCI-H1963FP5). In contrast, ADC-1 was not active in the two SCLC cell lines that were negative for SEZ6 mRNA (SW-1271 and SBC-5). Additionally, the non-targeting ADC-2 was inactive in all cell lines, confirming SEZ6-dependent cell killing specificity of ADC-1. All cell lines were sensitive to Example 1G, as indicated by sub- to low-nM IC₅₀. These data illustrate the SEZ6-specific activity of the ADC-1 in SCLC cell lines that expressed SEZ6.

**Table 2. SEZ6 Expression and Cell Viability in vitro**

| **SCLC cell line** | | | | |
|---|---|---|---|---|
| | **SEZ6 expression^{a}** | **ADC-1^{b} (IC₅₀ - nM)** | **ADC-2^{b} (IC₅₀ - nM)** | **Example 1G^{c} (IC₅₀ - nM)** |
| NCI-H524 | 84.32 | 0.33 (0.17 - 0.69) | 37.19 (17.38 - 78.92) | 1.68 (1.03 - 2.79) |
| NCI-H526 | 54.34 | 0.10 (0.07 - 0.12) | 55.13 (35.47 - 96.63) | 0.20 (0.19 - 0.21) |
| COR-L95 | 61.89 | 0.33 (0.19 - 0.58) | 84.81 (32.69 - 100) | 5.71 (3.10 - 11.68) |
| | | 0.29 (0.16 - 0.52) | | |
| NCI-H146 | 51.53 | 0.05 (0.04 - 0.07) | 48.39 (29.62 - 84.10) | 21.61 (12.12 - 41.58) |
| NCI-H1963 | 27.15 | 4.63 (2.84 - 8.03) | >100 (nd) | 6.28 (4.59 - 8.69) |
| SW-1271 | 0.01 | >100 (nd) | >100 (nd) | 2.49 (1.64 - 3.85) |
| SBC-5 | 0 | >100 (nd) | >100 (nd) | 1.26 (0.63 - 2.60) |

| | | | | |
|---|---|---|---|---|
| nd = not determined due to lack of activity ^{a} Fragment per kilobase of transcript per million mapped reads (FPKM) of SEZ6 as determined by the Cancer Cell Line Encyclopedia (CCLE), a publicly available database. ^{b}IC₅₀ values in nanomolar (nM) representing 50% growth inhibition compared to vehicle control (0%) and staurosporine (100%) treatments at 10 days, with the 95% confidence interval for duplicate titrations indicated in parentheses. ^{c} IC₅₀ values in nanomolar (nM) representing 50% cell growth inhibition compared to vehicle control (0%) and staurosporine (100%) treatments at 3 days, with the 95% confidence interval for duplicate titrations indicated in parentheses. | | | | |

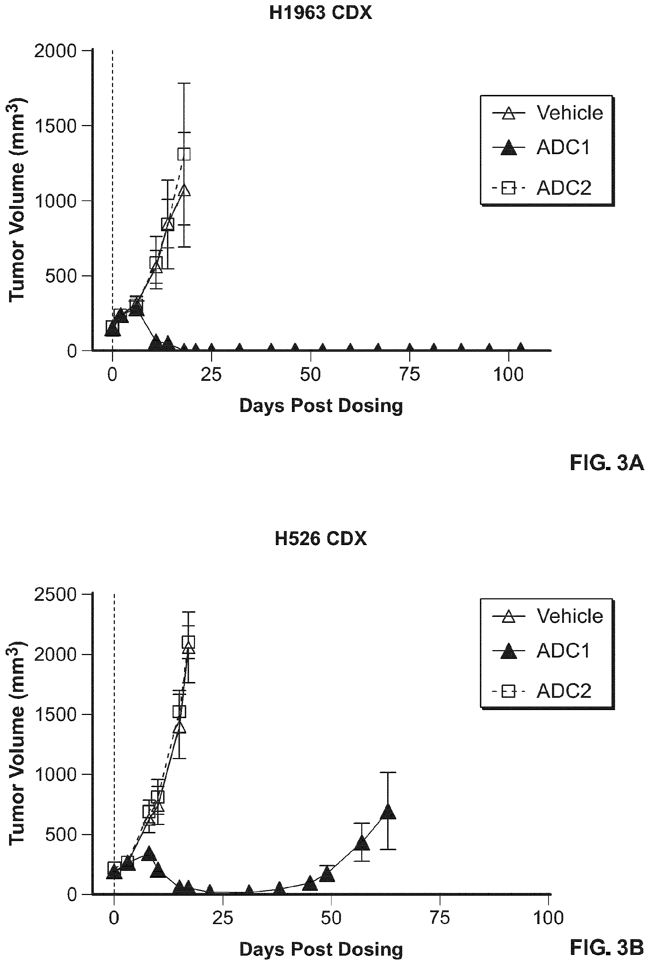

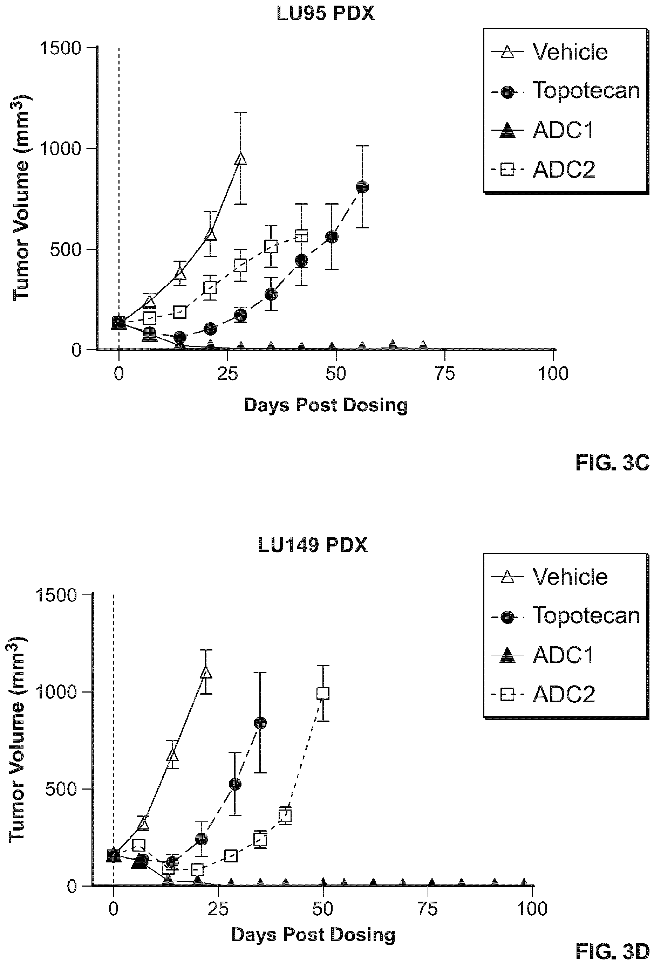

### Example 7: ADC-1 Inhibits the Growth of Cancer Xenografts In Vivo FIGS. 3A and 3B: Mouse CDXModels

Adult female SCID-bg (Fox Chase SCID Beige Mouse) or SCID CB.17 (Fox Chase SCID Mouse) mice (approximately 22 g) were obtained from Charles River (Wilmington, MA). The mice were housed in groups often in solid bottom Plexiglas cages equipped with constant air circulation in a facility approved by Association for Assessment and Accreditation of Laboratory Animal Care International (AAALAC). Animals were tested in the light phase of a 12 hour light: dark cycle.

NCI-H69 ("H69"), NCI-H1963 ("H1963"), and NCI-H526 ("H526") cells were obtained from the American Type Culture Collection (ATCC, Manassas, VA). The cells were maintained in 10% RPMI-1640 media. Cell culture media was obtained from Invitrogen (Carlsbad, CA) and supplemented with 10 % fetal bovine serum (Hyclone, Logan, UT).

Five million cells were inoculated subcutaneously into the right flank of the mice on day 0. The injection volume was 0.1 mL composed of a 1:1 mixture of culture medium and matrigel (BD, Franklin Lakes, NJ). Tumors were size matched at approximately 200 mm³. Therapy began 24 hours after size matching the tumors. There were four treatment groups: (1) vehicle; (2) ADC-1; (3) ADC-2; and (4) topotecan (ADC-1, ADC-2, and topotecan were formulated in vehicle). Each was administered once a day. Tumor volume was calculated two to three times weekly. Measurements of the length (L) and width (W) of the tumor were taken via electronic caliper and the volume was calculated according to the following equation: V = L × W²/2.

### FIGS. 3C-3D: Mouse PDXModels

SCLC patient-derived xenograft (PDX) models were established from stage IV tumors as previously described (Anderson, et al. 2015 PLoS One; PMID: 25955027), and passaged in mice to generate sufficient cell numbers to inject single cell suspensions subcutaneously in lower right mammary fat pads of NOD/SCID immune compromised mice for an efficacy study. Once tumor cells were injected, tumor volumes were collected using a caliper to measure the length and width, and tumor volume was calculated using the formula of an ellipsoid (volume = length × width × width × 0.5, where the width is shorter than the length; units: mm³). After tumors reached a range of 90 to 300 mm³, mice were randomized into treatment groups of five to ten mice per group, where each group had approximately the same average tumor volume. Randomized treatment groups of mice received the following: vehicle as a single intraperitoneal (ip) dose; topotecan standard of care (Hospira; 0.5 mg/kg once daily for 5 total doses administered ip; formulated with 0.9% saline); or ADCs (ADC-1 or ADC-2; formulated in vehicle) each administered as a single ip dose. Efficacy was measured by monitoring tumor volume over time, and data were plotted in GraphPad Prism.

Parameters of amplitude (TGIₘₐₓ) and durability (TGD) of therapeutic response were used to refer to the efficacy of the drug. TGIₘₐₓ is the maximum tumor growth inhibition during the experiment. Tumor growth inhibition (TGI) was calculated by 100^{∗}1-Tᵥ/Cᵥ) where Tᵥ and Cᵥ are the mean tumor volumes of the treated and control groups, respectively. TGD or tumor growth delay was the extended time of a treated tumor needed to reach a volume of 1 cm³ relative to the control group. TGD was calculated by 100^{∗}(Tₜ/Cₜ-1) where Tₜ and Ct are the median time periods to reach 1 cm³ of the treated and control groups, respectively.

### Results:

When administered as single agent in H1963 tumor bearing mice, ADC-1 inhibited tumor growth significantly, with (TGIₘₐₓ = 94%, p =0.002) and caused a durable response with a TGD of >472%. In the H526 cell line, ADC-1 inhibitor inhibited tumor growth significantly, with (TGIₘₐₓ =97.4%, p=<0.001 and durable response with a TGD of 336.7% (Figure 3A and Figure 3B).

ADC-1 treatment generated durable response (no detectable disease) in (1) cell line xenograft (CDX; H1963) and (2) patient-derived xenograft (PDX; LU95 and LU149) models. Strong efficacy (no measurable disease for > 30 days) was observed in the fast-growing and aggressive H526 CDX model, where vehicle treated tumors grew to >2000 mm³ in 17 days. The efficacy response was SEZ6 target specific, as there was little (modest to no tumor growth delay) with non-targeting ADC-2. ADC-1 was more efficacious than the second line standard of care for SCLC, topotecan, which was administered as one cycle at a dose and regimen to approximate the human clinical exposure (Figure 3A-D).

| **SEQUENCE LISTING TABLE** | | |
|---|---|---|
| SEQ ID NO: | Description | Sequence |
| 11 | Nucleic acid sequence encoding Ab heavy chain protein sequence including introns | atggaatggtcctgggtgttcctgttcttcctgtccgtgaccaccg gtgtgcactccgaagtccaactcgtccaatccggtgccgaagtg aaaaagcctggggaatccctgaagatcagctgcaagggatccg gttactcgttcacctcctcctggattaactgggtccggcagatgcc cggaaagggactggagtggatgggcagaatctatccgggcga aggggacactaattacaacggaaacttcgagggccaggtcacc atttcggccgataagagcatctcaaccgcgtacttgcagtggtca agcctgaaggcttccgacaccgccatgtactactgtactcgcgg ccttgtgatggactactggggacagggaactctcgtgaccgtgt cgtccgcctctaccaagggcccttccgtgttccctctggccccct cgagcaagagcacctctgggggcacagcggccctgggctgcc tggtcaaggactacttccccgagccggtgacggtgtcgtggaac tcaggcgccctgaccagcggcgtgcacaccttcccggctgtcct acagtcctcaggactctactccctcagcagcgtggtgaccgtgc cctccagcagcttgggcacccagacctacatctgcaacgtgaat cacaagcccagcaacaccaaggtggacaagaaagttggtgag aggccagcacagggagggagggtgtctgctggaagccaggct cagcgctcctgcctggacgcatcccggctatgcagccccagtc cagggcagcaaggcaggccccgtctgcctcttcacccggagg cctctgcccgccccactcatgctcagggagagggtcttctggctt tttccccaggctctgggcaggcacaggctaggtgcccctaaccc aggccctgcacacaaaggggcaggtgctgggctcagacctgc caagagccatatccgggaggaccctgcccctgacctaagccca ccccaaaggccaaactctccactccctcagctcggacaccttctc tcctcccagattccagtaactcccaatcttctctctgcagagccca aatctagtgacaaaactcacacatgcccaccgtgcccaggtaag ccagcccaggcctcgccctccagctcaaggcgggacaggtgc |
| | | cctagagtagcctgcatccagggacaggccccagccgggtgct gacacgtccacctccatctcttcctcagcacctgaactcctgggg ggaccgtcagtcttcctcttccccccaaaacccaaggacaccct catgatctcccggacccctgaggtcacatgcgtggtggtggacg tgagccacgaagaccctgaggtcaagttcaactggtacgtggac ggcgtggaggtgcataatgccaagacaaagccgcgggagga gcagtacaacagcacgtaccgtgtggtcagcgtcctcaccgtcc tgcaccaggactggctgaatggcaaggagtacaagtgcaaggt ctccaacaaagccctcccagcccccatcgagaaaaccatctcca aagccaaaggtgggacccgtggggtgcgagggccacatgga cagaggccggctcggcccaccctctgccctgagagtgaccgct gtaccaacctctgtccctacagggcagccccgagaaccacagg tgtacaccctgcccccatcccgggatgagctgaccaagaacca ggtcagcctgacctgcctggtcaaaggcttctatcccagcgacat cgccgtggagtgggagagcaatgggcagccggagaacaacta caagaccacgcctcccgtgctggactccgacggctccttcttcct ctacagcaagctcaccgtggacaagagcaggtggcagcaggg gaacgtcttctcatgctccgtgatgcatgaggctctgcacaacca ctacacgcagaagagcctctccctgtctccgggttga |
| 12 | Nucleic acid sequence encoding Ab light chain protein sequence | atggagacagacacactcctgctatgggtactgctgctctgggtt cccgggtccactggtgaaatcgtgttgacccagtcccccgctac cctgtcactgagccccggagaacgcgcgactctgtcctgccgg gcatcccagtccgtggactacaacggaatctcctacatgcactg gtatcagcaaaagccaggccaagccccgagactgctcatctac gccgcctcgaacgtgcagagcggtattccggcgcggttctccg gctcgggcagcggaaccgattttaccctcactatctcgtcacttg aacctgaggacttcgccgtgtactactgccagcagtccattgag |
| | | gacccgcctactttcggggggggaaccaaagtcgagatcaagc ggactgtggctgcaccaagtgtcttcatcttcccgccatctgatga gcagttgaaatctggaactgcctctgttgtgtgcctgctgaataac ttctatcccagagaggccaaagtacagtggaaggtggataacgc cctccaatcgggtaactcccaggagagtgtcacagagcaggac agcaaggacagcacctacagcctcagcagcaccctgacgctg agcaaagcagactacgagaaacacaaagtctacgcctgcgaa gtcacccatcagggcctgagctcgcccgtcacaaagagcttcaa caggggagagtgttga |

## Claims

1. An anti-SEZ6 antibody drug conjugate of formula (IV): 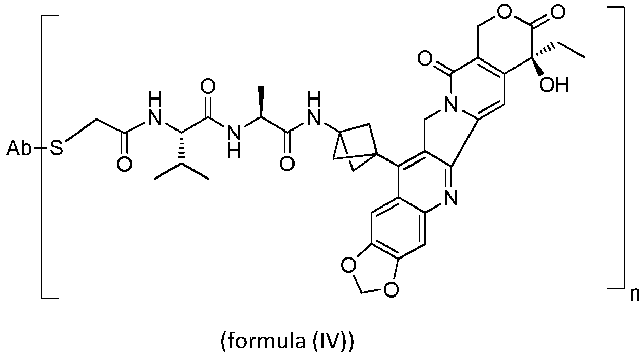 wherein n is an integer from 1 to 10, and wherein Ab is an anti-SEZ6 antibody comprising a heavy chain variable region comprising a CDR-H1, a CDR-H2, and a CDR-H3; and a light chain variable region comprising a CDR-L1, a CDR-L2, and a CDR-L3, wherein
CDR-H1 has the amino acid sequence shown as SEQ ID NO: 3,
CDR-H2 has the amino acid sequence shown as SEQ ID NO: 4,
CDR-H3 has the amino acid sequence shown as SEQ ID NO: 5;
CDR-L1 has the amino acid sequence shown as SEQ ID NO: 6,
CDR-L2 has the amino acid sequence shown as SEQ ID NO: 7, and
CDR-L3 has the amino acid sequence shown as SEQ ID NO: 8.

2. The anti-SEZ6 antibody drug conjugate according to claim 1, wherein the antibody is an IgG1 antibody.

3. The anti-SEZ6 antibody drug conjugate according to claim 1, wherein the antibody comprises
(a) a heavy chain variable region having the amino acid sequence shown as SEQ ID NO: 1 and
(b) a light chain variable region having the amino acid sequence shown as SEQ ID NO: 2.

4. The anti-SEZ6 antibody drug conjugate according to claim 1, wherein the antibody comprises
(a) a heavy chain having the amino acid shown as SEQ ID NO: 9 and
(b) a light chain having the amino acid sequence shown as SEQ ID NO: 10.

5. An anti-SEZ6 antibody drug conjugate of formula (IV): 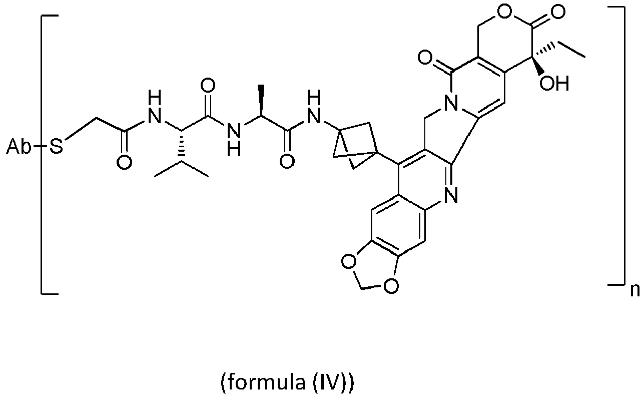 wherein n is an integer from 1 to 10, and wherein Ab is an anti-SEZ6 antibody comprising: two heavy chains each having an amino acid sequence shown as SEQ ID NO: 9 and two light chains each having an amino acid sequence shown as SEQ ID NO: 10.

6. The anti-SEZ6 antibody drug conjugate according to any one of claims 1-5, wherein n is 6.

7. The anti-SEZ6 antibody drug conjugate according to any one of claims 1-5, wherein n is 2.

8. The anti-SEZ6 antibody drug conjugate according to any one of claims 1-5, wherein n is 4.

9. The anti-SEZ6 antibody drug conjugate according to any one of claims 1-5, wherein n is 8.

10. The anti-SEZ6 antibody drug conjugate according to any one of claims 1-5, wherein n is 10.

11. A composition comprising a therapeutically effective amount of an antibody drug conjugate according to any of claims 1-5, wherein the predominant species of the antibody drug conjugate in the composition has an n of 6.

12. A pharmaceutical composition comprising a therapeutically effective amount of an antibody drug conjugate according to any of claims 1-5 and a pharmaceutically acceptable excipient, wherein the composition has a DAR of 6 or about 6.

13. A method of treating small cell lung cancer, the method comprising administering a therapeutically effective amount of an antibody drug conjugate according to any one of claims 1-5 to a patient in need thereof.

14. A method of treating small cell lung cancer, the method comprising administering a therapeutically effective amount of the pharmaceutical composition of claim 12 to a patient in need thereof.
